# EUROPÄISCHE PATENTANMELDUNG

(11) **EP 1 593 401 A1**
(43) Veröffentlichungstag der Anmeldung: **09.11.2005**
(21) Anmeldenummer: 05007955.7
(22) Anmeldetag: 12.04.2005
(51) Int. Cl.: A61M 1/00, F16L 5/06

(54) **Sauger für die Chirurgie**

(30) Priorität: 03.05.2004 DE 102004021676
(71) Anmelder: Fehling AG, 4153 Reinach BL 1 (CH)
(72) Erfinder: Hoell, Thomas, Dr., 06193 Gutenberg (DE); Fehling, Guido, 63791 Karlstein (DE)
(74) Vertreter: Mussgnug, Bernd

(57) **Zusammenfassung**

Ein Sauger für die Chirurgie besteht aus einem als Handstück (10) ausgebildetem Griff und einem als separates Teil ausgebildeten Saugrohr (16). Das Saugrohr (16) ist in seiner Länge verstellbar, indem es axial in das Handstück (10) einschiebbar ist. In seiner eingestellten axialen Position wird das Saugrohr (16) durch eine Dichtung (20) reibschlüssig fixiert und abgedichtet.

## Beschreibung

Die Erfindung betrifft einen Sauger für die Chirurgie gemäß dem Oberbegriff des Anspruchs 1.

In der Chirurgie sind Sauger ein wesentliches Instrument, um Blut und andere Flüssigkeiten aus dem Operationsfeld abzusaugen. Der Sauger wird hierzu über einen flexiblen Schlauch an ein Unterdruck-System angeschlossen. Die Sauger weisen ein Saugrohr auf, welches in eine distale Spitze ausläuft. Am proximalen Ende des Saugrohres ist ein Griff angeformt, um den Sauger zu halten und in dem Operationsfeld zu dirigieren. Die Länge und der Durchmesser des Saugrohres sowie die Form der Spitze sind dem jeweiligen Verwendungszweck angepasst. Die Länge kann zwischen 5 und 60 cm liegen. Der Durchmesser kann bis zu 30 mm betragen. Bei neurochirurgischen Operationen, insbesondere im Gehirn, werden bei Arbeit unter dem Operationsmikroskop auch sehr feine Saugrohre bis hinunter zu einem Durchmesser von ca. 1,5 mm verwendet.

In der Neurochirurgie werden auch Sauger verwendet, die im Bereich des Griffes eine Luftöffnung aufweisen, die während des Saugvorgangs mit dem Daumen verschlossen wird. Wird der Daumen von dieser Luftöffnung abgehoben, zieht der Sauger durch diese Luftöffnung Luft an und die Saugwirkung an der Spitze lässt entsprechend nach. Wird während der Operation in unerwünschter Weise Gewebe an der Saugerspitze angesaugt, so kann durch Freigabe der seitlichen Luftöffnung der Sog an der Saugerspitze soweit reduziert werden, dass das angesaugte Gewebe wieder abfällt. Dies ist zum Beispiel wichtig, wenn im Bereich der Hirnnerven und -gefäße operiert wird, die sehr empfindlich sind und durch das Ansaugen mit der Saugerspitze bereits zerstört oder herausgezogen werden können.

Während der Operation legt der Operateur seine Hand auf einer festen Unterlage in unmittelbarer Nähe der Operationsöffnung auf, um ein Zittern der Hand und eine Ermüdung zu vermeiden. Mit Fortschreiten des operativen Eingriffs in die Tiefe, wie dies beispielsweise bei Operationen am Kopf in der Regel der Fall ist, benötigt der Operateur daher zunehmend unterschiedlich lange Saugrohre, weil der Abstand zwischen der Hand und der tiefsten Stelle des Operationsfeldes, an welcher die Saugerspitze zum Einsatz kommt, zunimmt. Bei den herkömmlichen chirurgischen Saugern werden daher Sauger unterschiedlicher Länge und unterschiedlichen Durchmessers am Operationstisch bereit gehalten, die der Operateur jeweils entsprechend dem Fortgang der Operation einsetzt. Da die Zahl der unterschiedlichen Sauger aus ökonomischen und praktischen Gründen beschränkt ist, treten daher immer wieder Operationsumstände und -geometrien auf, in welchen der Operateur mit einer unangepassten Saugerlänge arbeiten muss.

Der Erfindung liegt die Aufgabe zugrunde, diesem Missstand abzuhelfen und einen Sauger zur Verfügung zu stellen, der eine Anpassung der Länge des Saugrohres an die Operationsgegebenheiten zulässt.

Diese Aufgabe wird erfindungsgemäß gelöst durch einen Sauger mit den Merkmalen des Anspruchs 1.

Vorteilhafte Ausführungen und Weiterbildungen der Erfindung sind in den Unteransprüchen angegeben.

Erfindungsgemäß sind der Griff und das Saugrohr des Saugers als getrennte Teile hergestellt. Der Griff besteht aus einem Handstück mit einem axial durchgehenden Innenkanal. Das Saugrohr wird in das distale Ende des Handstückes eingesetzt und kann in seiner wirksamen Arbeitslänge dadurch variiert und eingestellt werden, dass das Saugrohr axial unterschiedlich weit in das Handstück eingeschoben wird. In seiner jeweiligen eingestellten axialen Position und Drehstellung wird das Saugrohr in dem Handstück kraftschlüssig fixiert. Die kraftschlüssige Fixierung lässt eine beliebige axiale Positionierung des Saugrohres in dem Handstück zu, sodass die wirksame Länge des Saugrohres und seine Drehstellung in Bezug auf das Handstück optimal den jeweiligen Operationsbedingungen angepasst werden kann und auch im Verlauf der Operation nachgestellt werden kann. Das Saugrohr ist an seinem Außenumfang in dem Handstück abgedichtet, sodass der Unterdruck, der über den an das proximale Ende des Handstückes angeschlossenen Schlauch zugeführt wird, das Saugrohr beaufschlagt. In einer vorteilhaften Ausführung wird die Abdichtung des Saugrohres in dem Handstück durch eine Dichtung bewirkt, die in Form einer hohlzylindrischen elastischen Buchse im distalen Ende des Handstückes aufgenommen ist. Diese Dichtung dient dabei gleichzeitig zur kraftschlüssigen Fixierung des Saugrohres in seiner jeweiligen axialen Position. Vorzugsweise kann eine die Dichtung bildende elastische Buchse durch eine auf das distale Ende des Handstückes aufschraubbare Überwurfmutter axial gestaucht werden, sodass sich ihr Innendurchmesser verengt und eine stabile axiale und rotatorische Fixierung des Saugrohres bewirkt.

Zweckmäßig ist der Innendurchmesser des Innenkanals des Handstückes zumindest im proximalen Bereich gegenüber dem Außendurchmesser des Saugrohres aufgeweitet, sodass ein Luftspalt im Inneren des Handstückes frei bleibt. Eine durch den Finger des Operateurs verschließbare Luftöffnung führt radial in diesen Luftspalt, sodass ein Belüften möglich ist, wenn die Saugwirkung an der Saugspitze reduziert werden soll.

Die getrennte Ausführung des Griffes und des Saugrohres ermöglicht eine wirtschaftliche Ausbildung und Nutzung des Saugers. Es können mehrere Saugrohre austauschbar mit demselben Handstück verwendet werden. Es können beispielsweise gebrauchte und gegebenenfalls beschädigte Saugrohre durch neue und sterile Saugrohre ersetzt werden. Es können Saugrohre mit unterschiedlich geformten Saugspitzen verwendet werden. Es können auch Saugrohre mit unterschiedlichem Durchmesser verwendet werden, wobei geringe Differenzen des Außendurchmessers des Saugrohres durch die axial gestauchte Dichtung aufgenommen werden können. Bei größeren Differenzen des Außendurchmessers können Dichtungen mit entsprechendem unterschiedlichen Innendurchmesser eingesetzt werden. Dabei erleichtert eine Farbkodierung der Dichtungen und der Saugrohre die einfache und zuverlässige Zuordnung. Die Dichtung ist dabei vorzugsweise als axial langgestreckte Buchse ausgebildet, die das Saugrohr schlauchförmig umschließt. Die Dichtung stützt dadurch das Saugrohr auch gegen ein Verkippen seiner Achse in dem Handstück ab, was insbesondere wichtig ist, wenn das Saugrohr ein radiales Spiel in dem Innenkanal des Handstückes aufweist.

Das Handstück kann kostengünstig aus Kunststoff gefertigt sein. Es ist auch eine Herstellung aus Metall, insbesondere Edelstahl, möglich, was besonders für ein mehrfach verwendbares sterilisierbares Handstück vorteilhaft ist.

Die Saugrohre können ebenfalls kostengünstig aus Kunststoff gefertigt sein. Für manche Verwendungszwecke ist es vorteilhaft, das Saugrohr aus einem verformbaren Metall herzustellen, sodass das Saugrohr während der Operation in eine zweckmäßige Form gebogen werden kann. Da in der Regel im Verlauf der Operation die erforderliche Länge des Saugrohres zunimmt, werden bei der Verlängerung des Saugrohres jeweils unverbogene Saugrohrlängen aus dem Handstück herausgezogen, sodass ein Nachbiegen zur Anpassung an den Operationsfortgang möglich ist. Die Abdichtung erfolgt dabei immer in einem noch unverformten axialen Bereich des Saugrohres. Dabei kann das Saugrohr auch aus einer Memory-Legierung gefertigt sein, sodass das während der Operation verbogene Saugrohr bei der anschließenden Hitze-Sterilisation wieder seine ursprüngliche unverformte Gestalt annimmt.

Für den Anschluss des Saugers an das Unterdruck-System wird im Allgemeinen ein Schlauch verwendet, der semirigide ist, um den Unterdruck aufnehmen zu können. Die Steifigkeit des Schlauches hat zur Folge, dass das Innenlumen des Schlauches über eine gewisse axiale Länge fluchtend den Innenkanal des Handstückes fortsetzt. Das Saugrohr kann daher mit seinem proximalen Ende über das proximale Ende des Handstückes hinaus in den Schlauch eingeschoben werden. Dadurch ist eine große Verstelllänge des Saugrohres möglich, ohne dass das Handstück eine entsprechend große axiale Länge aufweisen muss.

Der ökonomische Vorteil der Erfindung besteht darin, dass nicht eine große Anzahl verschiedener Sauger am Operationstisch bereit gehalten werden muss. Es genügen ein Handstück oder einige wenige Handstücke für die erforderliche Anzahl von Saugrohren. Dabei kann auch die Zahl der Saugrohre reduziert werden, da deren Länge verstellbar ist. Die Anzahl der benötigten kostenaufwändigen Handstücke wird deutlich reduziert, wobei die vergleichsweise kostengünstigen Saugrohre in größeren Stückzahlen zum Einsatz kommen. Aus hygienischen Gründen ist es vorteilhaft, dass die kostengünstigen Saugrohre die Verwendung als Einmalartikel ermöglichen.

Schließlich verbessert die stufenlose axiale Längenverstellung der Saugrohre die Brauchbarkeit des Saugers für die Erfordernisse des Operateurs.

Im Folgenden wird die Erfindung anhand eines in der Zeichnung dargestellten Ausführungsbeispiels näher erläutert. Es zeigen
- Fig. 1: einen Axialschnitt durch einen Sauger und
- Fig. 2: eine Draufsicht auf die Luftöffnung des Saugers.

Der Sauger weist einen Griff auf, der aus einem Handstück 10 besteht. Das Handstück 10 hat die Grundform eines Rohres und ist ergonomisch optimiert für die Führung durch die Hand des Operateurs gestaltet. Das Handstück 10 ist in axialer Richtung von einem Innenkanal 12 durchsetzt, der insbesondere kreisförmigen Querschnitt aufweist. Am proximalen Ende des Handstückes 10 sind Anschlussmittel 14 zum Befestigen eines Schlauches für die Zuleitung des Unterdruckes angeformt. Diese Anschlussmittel können als Schlaucholive, Schraubanschluss oder in sonstiger an sich bekannter Weise ausgebildet sein.

Ein Saugrohr 16 kann mit seinem proximalen Ende von der distalen Seite her in den Innenkanal 12 des Handstückes 10 eingeführt werden. Das Saugrohr 16 weist einen Außendurchmesser auf, der geringfügig kleiner ist als der Innendurchmesser des Innenkanals 12, so dass das Saugrohr 16 in dem Handstück 10 axial verschiebbar und im wesentlichen gegen ein Verkippen radial abgestützt ist. Am distalen Ende kann das Saugrohr zu einer Saugspitze 18 geformt sein.

Am distalen Ende des Handstückes 10 ist der Innenkanal 12 im Durchmesser zu einer Aufnahme 24 erweitert. In diese Aufnahme 24 ist eine Dichtung 20 eingesetzt. Die Dichtung 20 hat die Form einer länglichen hohlzylindrischen Buchse und besteht aus einem elastisch verformbaren Kunststoff. Der Außendurchmesser der Dichtung 20 entspricht dem Innendurchmesser der Aufnahme 24, während der Innendurchmesser der Dichtung 20 dem Außendurchmesser des Saugrohres 16 entspricht. Die Dichtung 20 stützt sich mit ihrem proximalen Ende axial an einer Innenschulter ab, die durch das proximale Ende der Aufnahme 24 gebildet ist. Das distale Ende der Dichtung 20 ragt etwas über das distale Ende des Handstückes 10 hinaus. Auf ein Außengewinde am distalen Ende des Handstückes 10 ist eine Überwurfmutter 22 aufschraubbar, die von dem Saugrohr 16 frei durchsetzt wird.

Wird die Überwurfmutter 22 gelockert, d. h. in distaler Richtung geschraubt, so ist die Dichtung 20 entspannt. Das Saugrohr 16 kann daher axial in der Dichtung 20 und damit in dem Innenkanal des Handstückes 10 verschoben werden, wobei die Dichtung 20 nur eine geringe Reibung bewirkt. Ist das Saugrohr 16 in der gewünschten axialen Lage in dem Handstück 10 positioniert, so wird die Überwurfmutter 22 in proximaler Richtung auf dem Handstück 10 aufgeschraubt. Dabei drückt die Überwurfmutter 22 auf das distal überstehende Ende der Dichtung 20 und staucht die Dichtung 20 in der Aufnahme 24 axial zusammen. Dadurch vergrößert sich die Wanddicke der Dichtung 20 und die Dichtung 20 legt sich abdichtend und unter radialem Druck an der Innenwand der Aufnahme 24 und an dem Außenumfang des Saugrohres 16 an. Die Dichtung 20 dichtet auf diese Weise das Saugrohr 16 luftdicht in dem Handstück 10 ab und fixiert das Saugrohr 16 kraftschlüssig gegen axiale Verschiebung und Verdrehung in dem Handstück 10.

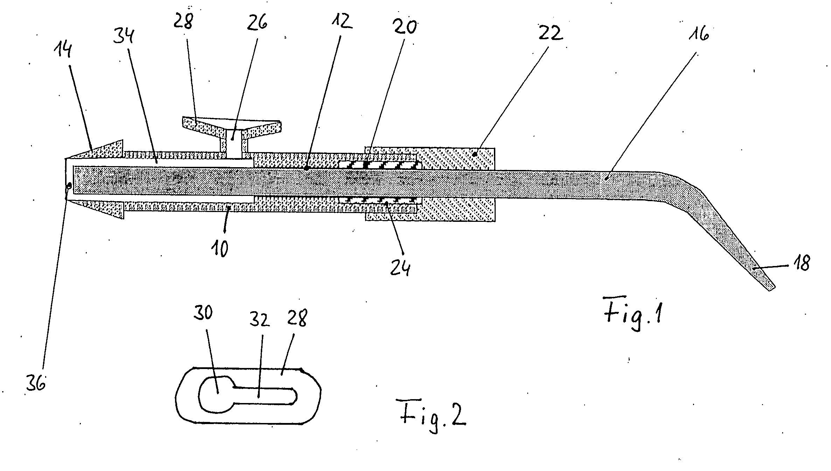

Proximal hinter der Dichtung 20 und von dieser axial beabstandet ist die Wandung des Handstückes 10 durch eine Luftöffnung 26 durchbrochen. An der Außenseite des Handstückes 10 ist das Austrittsende der Luftöffnung 26 von einer muldenförmigen Fingerplatte 28 umschlossen. Der Querschnitt der Luftöffnung 26 hat, wie in Fig. 2 gezeigt ist, die Form eines Schlüsselloches mit einem kreisrunden Abschnitt 30 größeren Durchmessers am proximalen Ende und einem langgestreckten schmalen Abschnitt 32, der sich in distaler Richtung erstreckt. Die Luftöffnung 26 mündet in einen axialen Luftspalt 34 zwischen der Innenwand des Innenkanals 12 und dem gegebenenfalls eingeschobenen Saugrohr 16, der über die Anschlussmittel 14 mit dem Schlauch und damit der Unterdruckquelle in Verbindung steht. Der Luftspalt 34 kann durch eine axiale Nut gebildet sein, die in der Innenwandung des Innenkanals 12 von dem proximalen Ende bis zu der Luftöffnung 26 führt. Vorzugsweise ist jedoch der Durchmesser des Innenkanals 12 vom proximalen Ende bis zu der Luftöffnung 26 erweitert, so dass der Luftspalt 34 als Ringspalt zwischen der Innenwand des Innenkanals 12 und dem Saugrohr 16 freibleibt. Der Operateur verschließt mit seinem auf die Fingerplatte 28 aufgesetzten Daumen die Luftöffnung 26, sodass der Unterdruck voll über das Saugrohr 16 an der Saugspitze 18 wirksam wird. Hebt der Operateur seinen Daumen leicht von der Fingerplatte 28 ab, so gibt er zunächst den schmalen Abschnitt 32 der Luftöffnung 26 frei. Durch die geringe Breite des schmalen Abschnittes 32 kann der Operateur daher fein dosiert Luft durch die Luftöffnung 26 eintreten lassen, um die Saugwirkung an der Saugspitze 18 feinfühlig zu reduzieren. Hebt der Operateur seinen Daumen vollständig von der Fingerplatte 28 ab, so gibt er auch den runden Abschnitt 30 frei, sodass Luft über einen großen Querschnitt der Luftöffnung 26 eintreten kann und an der Saugspitze 18 keine Saugwirkung mehr auftritt.

Ist das Saugrohr 16 als mehrfach verwendbares Saugrohr 16 aus Metall ausgebildet, so kann an dem proximalen Ende 36 des Saugrohres 16 ein Spülanschluss angebracht sein, z.B. ein Luer-Lock-Anschluss, so dass das Saugrohr 16 zur Reinigung durchgespült werden kann.

### Bezugszeichenliste

- 10: Handstück
- 12: Innenkanal
- 14: Anschlussmittel
- 16: Saugrohr
- 18: Saugspitze
- 20: Dichtung
- 22: Überwurfmutter
- 24: Aufnahme
- 26: Luftöffnung
- 28: Fingerplatte
- 30: runder Abschnitt
- 32: schmaler Abschnitt
- 34: Luftspalt
- 36: Spülanschluß

## Patentansprüche

1. Sauger für die Chirurgie, mit einem Griff, mit einem distal an dem Griff angeordneten Saugrohr und mit proximal an dem Griff angeordneten Anschlussmitteln für einen Schlauch,
**dadurch gekennzeichnet, dass**
der Griff aus einem von dem Saugrohr (16) getrennten Handstück (10) besteht, dass das Handstück (10) einen axial durchgehenden Innenkanal (12) aufweist und dass das Saugrohr (16) axial verschiebbar, kraftschlüssig fixierbar und abgedichtet in das distale Ende des Handstückes (10) einsetzbar ist und **dadurch** in einstellbarer Länge axial in den Innenkanal (12) hineinragt bzw. durch diesen hindurchragt.

2. Sauger nach Anspruch 1,
**dadurch gekennzeichnet , dass**
in das distale Ende des Handstückes (10) eine Dichtung (20) einsetzbar ist, die den Ringspalt zwischen dem Innenkanal (12) und dem Saugrohr (16) abdichtend verschließt und das Saugrohr (16) kraftschlüssig in dem Handstück (10) fixiert.

3. Sauger nach Anspruch 2,
**dadurch gekennzeichnet, dass**
die Dichtung (20) eine hohlzylindrische elastische Buchse ist, die in eine Aufnahme (24) des Innenkanals (12) eingesetzt ist und durch eine auf das distale Ende des Handstückes (10) aufschraubbare Überwurfmutter (22) gehalten und axial gestaucht wird.

4. Sauger nach einem der vorhergehenden Ansprüche,
**dadurch gekennzeichnet, dass**
der Innenkanals (12) im proximalen Endbereich einen gegen das proximale Ende offenen Luftspalt (34) bildet und dass eine Luftöffnung (26) durch die Wandung des Handstückes (10) in diesen Luftspalt (34) führt.

5. Sauger nach Anspruch 4,
**dadurch gekennzeichnet, dass**
die Luftöffnung (26) an ihrem äußeren Austrittsende mit einer Fingerplatte (28) ausgebildet ist.

6. Sauger nach Anspruch 5,
**dadurch gekennzeichnet, dass**
die Luftöffnung (26) einen schlüssellochförmigen Querschnitt aufweist, dessen schmaler Abschnitt (32) nach distal gerichtet ist.

7. Sauger nach einem der Ansprüche 1-6,
**dadurch gekennzeichnet, dass**
das Saugrohr (16) aus Kunststoff besteht.

8. Sauger nach einem der Ansprüche 1-6,
**dadurch gekennzeichnet, dass**
das Saugrohr (16) aus Metall besteht.

9. Sauger nach Anspruch 8,
**dadurch gekennzeichnet, dass**
das Saugrohr (16) aus einem verformbaren Metall besteht.

10. Saugrohr nach Anspruch 9,
**dadurch gekennzeichnet, dass**
das Saugrohr (16) aus einer Memory-Legierung besteht.

11. Sauger nach einem der Ansprüche 8-10,
**dadurch gekennzeichnet, dass**
das proximale Ende des Saugrohres (16) mit einem Spülanschluss (36), insbesondere einem Luer-Lock-Anschluss, ausgebildet ist.
